# EUROPEAN PATENT APPLICATION

(11) **EP 4 846 991 A1**
(43) Date of publication of application: **02.12.2026**
(21) Application number: 26154953.9
(22) Date of filing: 29.01.2026
(51) Int. Cl.: A61F 13/49, A61F 13/496

(54) **ABSORBENT ARTICLE WITH IMPROVED SHAPE**

(30) Priority: 30.05.2025 EP 25180003; 08.08.2025 EP 25194852; 24.11.2025 EP 25217890
(71) Applicant: Ontex BV, 9255 Buggenhout (BE); Ontex Group NV, 9320 Erembodegem (BE)
(72) Inventor: Van Sande, Nathalie, 9688 Maarkedal (BE)
(74) Representative: Cobbaert, Dries

(57) **Abstract**

The present disclosure relates to an absorbent article, such as an incontinence pant, designed for improved fit and leakage prevention. The article comprises a front waist belt, a rear waist belt, and an absorbent insert. The rear leg edge includes a convex curved segment, and the absorbent core has a greater width in the rear than in the front or a greater width in the front compared to the rear of the absorbent core. This configuration optimizes the geometry of the leg edges and core to enhance comfort and containment depending on the anatomy of the wearer.

## Description

### TECHNICAL FIELD

The present invention relates to absorbent articles, more specifically to absorbent articles for personal hygiene, such as diapers or incontinence underpants, that include an absorbent core and elasticized waist and leg openings.

### BACKGROUND

Absorbent articles, such as diapers and incontinence pants, are well known in the art. These articles are designed to absorb and contain bodily fluids, such as urine and feces, to prevent soiling of clothing or other surfaces. Many absorbent articles comprise a front waist region, a back waist region, and a crotch region extending between the front and back waist regions, as well as an absorbent core for retaining fluids.

Conventional absorbent articles often employ elastic elements in the waist and leg regions to improve fit and prevent leakage. For example, elastic strands or films may be incorporated into the waist belts and leg cuffs to provide a snug and secure fit against the wearer's body. However, achieving a balance between effective containment and comfortable fit can be challenging.

EP1603502B1 discloses an absorbent garment with improved fit and comfort, where the garment includes a body-facing layer, a back sheet, and an absorbent core. The absorbent core has a front waist portion, a crotch portion, and a back waist portion. The back waist portion has a pair of concave arcuate leg cuffs. The elastic gathering means are placed along the leg cuffs. A drawback of this absorbent garment is that it can lack optimal fit and may not effectively prevent leakage, particularly in the rear leg area.

US20080275415A1 describes a disposable absorbent article including an absorbent core having a front waist region, a back waist region, and a crotch region. The absorbent core has a greater absorbent capacity in the back waist region than in the front waist region, and the back waist region has a greater width than the front waist region. A drawback of this absorbent article is that it may not provide an optimal fit around the legs, potentially leading to leakage.

Absorbent articles with elastic waist belts and shaped absorbent cores are known. These features aim to provide a better fit and prevent leakage. However, there remains a need for further improvements in the design of absorbent articles, particularly in the shaping of the leg edges and the absorbent core, to enhance comfort and performance.

Therefore, there is a need to provide an absorbent article with improved fit and leakage prevention characteristics, which can be achieved by optimizing the geometry of the leg edges and the absorbent core.

### SUMMARY

According to a first aspect of the disclosure, an absorbent article is preferably in the form of a pant, comprising: a front waist belt; a rear waist belt; an absorbent insert joined to the front and rear waist belts; said absorbent insert comprising a top sheet, a back sheet and an absorbent core, wherein said absorbent core comprises a front core portion, a middle core portion and a rear core portion; wherein the front and rear waist belts each comprise an outer nonwoven layer, an inner nonwoven layer, and an elastic material sandwiched between said outer and inner nonwoven layers; the front and rear waist belts joined together at a pair of side seams to form a waist opening with a waist edge extending in transverse direction; wherein said absorbent article further comprises two leg openings each being bordered by a leg edge having a front leg edge being located generally on the front waist belt, a rear leg edge being located generally on the rear waist belt and a crotch leg edge generally located between the front leg edge and the rear leg edge, characterised in that said rear leg edge comprising at least a first convex curved segment and said absorbent core has a first width and a second width, wherein said second width is larger than said first width. This may provide an improved fit and leakage prevention by optimizing the geometry of the leg edges and the absorbent core.

According to a preferred embodiment of the disclosure, an absorbent article has an absorbent core comprising an absorbent core crotch width being less than the first width and the second width. This may contribute to improved comfort and fit in the crotch region.

According to a preferred embodiment of the disclosure, an absorbent article has an absorbent core crotch width positioned in the middle core portion and measured at a point where the absorbent core is narrowest in the middle core portion. This may provide a more specific location for the reduced width, further enhancing comfort.

According to a preferred embodiment of the disclosure, an absorbent article has a front leg edge comprising at least a first concave curved segment. This may increase the freedom of movement and comfort of the subject.

According to a preferred embodiment of the disclosure, an absorbent article has at least a first concave curved segment with a first radius and at least a first convex curved segment with a second radius, wherein the second radius is larger than the first radius. Having a larger radius in the convex curved segment may contribute to improved fit and reduced leakage.

According to a preferred embodiment of the disclosure, an absorbent article has a first width positioned in a front core portion and measured at a point where the absorbent core is widest in the front core portion and a second width is positioned in a rear core portion and is measured at a point where the absorbent core is widest in the rear core portion. This may provide a more precise definition of the core shape, enhancing its functionality.

According to a preferred embodiment of the disclosure, an absorbent article has a second width positioned in a front core portion and measured at a point where the absorbent core is widest in the front core portion and a first width is positioned in a rear core portion and is measured at a point where the absorbent core is widest in the rear core portion. This may provide a more precise definition of the core shape, enhancing its functionality.

According to a preferred embodiment of the disclosure, an absorbent article has a ratio of the second radius and the second width is between about 1,5 and 7,5; more preferably between about 2 and 7; more preferably between about 3 and 6,5; more preferably between about 3,5 and 6; even more preferably between about 4 and 6. This specific range may enhance the convex curvature in the rear waist portions.

According to a preferred embodiment of the disclosure, an absorbent article has a rear leg profile width, measured according to the method disclosed herein, and a ratio of the rear leg profile width and the second width is between about 2 and 6, more preferably between about 2,5 and 5; most preferably between about 3 and 4. This may contribute to the overall fit and comfort of the absorbent article.

According to a preferred embodiment of the disclosure, an absorbent article has a ratio of the first radius and the second width between about 1,5 and 7,5; more preferably between about 1,5 and 6; more preferably between about 1,5 and 4; more preferably between about 1,75 and 2,5; even more preferably between about 1,8 and 2,25. This specific range may enhance the concave curvature in the front waist portions.

According to a preferred embodiment of the disclosure, an absorbent article has a ratio of the second width and the first width is between about 1,03 and 1,5; preferably between about 1,1 and 1,4; even more preferably between 1,15 and 1,3; most preferably between 1,18 and 1,25. This may provide a specific range for the width ratio, optimizing the balance between fit and absorbency.

According to a preferred embodiment of the disclosure, wherein the front waist belt has a width W4 and the rear waist belt has a width W5 and wherein the ratio of W4 and W2 is between about 3 and 10, preferably between about 3 and 9, more preferably between about 3 and 8, or wherein the ratio of W5 and W2 is between about 3 and 10, preferably between about 3 and 9, more preferably between about 3 and 8. This may provide for better fit and comfort in the front waist region or back waist region in relation to the positioning of the widest part of the absorbent core.

According to a preferred embodiment of the disclosure, an absorbent article has an elastic material selected from the group consisting of an elastic film, elastic strands, elastic fibers and combinations thereof. This may provide flexibility in material selection, allowing for different performance and cost characteristics.

According to a preferred embodiment of the disclosure, an absorbent article has an elastic material partially overlapping the rear core portion. This may further enhance the fit and containment in the rear of the article.

According to a preferred embodiment of the disclosure, an absorbent article has an elastic material partially overlapping the front core portion. This may further enhance the fit and containment in the front of the article.

According to a preferred embodiment of the disclosure, an absorbent article has a front waist belt comprising a first plurality of elastic strands and a rear waist belt comprising a second plurality of elastic strands, each of the first and second pluralities have an Average-Strand-Spacing from 0,25 mm to 4 mm as measured according to the test method set out herein. This may provide specific parameters for the elastic strands, optimizing the elastic properties of the waist belts.

According to a preferred embodiment of the disclosure, an absorbent article has each of the first and second pluralities of elastic strands with an Average-Dtex from 10 to 400 as measured according to the test method set out herein. This may provide additional specifications for the elastic strands, further optimizing the elastic properties of the waist belts.

According to a preferred embodiment of the disclosure, an absorbent article has at least a portion of each of the first and second pluralities of elastic strands with a Pressure-Under-Strand of from 689 to 6895 Pa (0,1 to 1 psi) as measured according to the test method set out herein. By defining the pressure under the strand a specific, consistent elastic force may be achieved.

According to a preferred embodiment of the disclosure, an absorbent article has each of the first and second plurality of elastic strands with an Average-Pre-Strain from 100% to 250% as measured according to the test method set out herein. This may contribute to the elastic properties that keep the article in place.

According to a preferred embodiment of the disclosure, an absorbent article has an Application-Force of from 900 gf to 1600 gf as measured according to the test method set out herein. This may contribute to a defined and controlled force with which the absorbent article adheres to the subject.

According to a preferred embodiment of the disclosure, the elastic material comprises elastic fibers comprising a thermoplastic elastomer and said fibers running substantially parallel to each other and oriented along a transversal axis (x). Advantageously, the elastic properties are derived directly from the engineered structure of the front and rear waist belts, rather than from separate elastic films or strands merely sandwiched between nonwoven layers. This integrated approach simplifies the overall belt construction and can lead to superior performance and tactile characteristics.

### BRIEF DESCRIPTION OF THE FIGURES

The disclosure can be better understood with reference to the following drawings and description. The components in the figures are not necessarily to scale, emphasis instead being placed upon illustrating various preferred embodiments of the disclosure.
Fig. 1 is a plan view of an unassembled and fully stretched absorbent article according to an embodiment of the present disclosure.

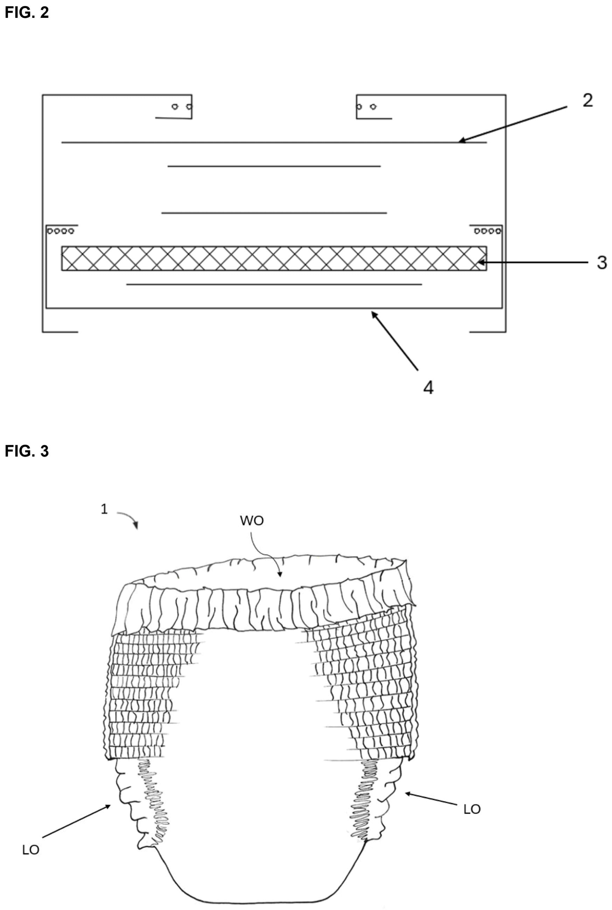

Fig. 2 is a plan view of an unassembled and fully stretched absorbent article according to an embodiment of the present disclosure, highlighting the elastified areas.
Fig. 3 is a front view of an absorbent article according to the disclosure, positioned on a mannequin.
Fig. 4 is a front view of an absorbent article which is not in accordance with the present disclosure, positioned on a mannequin.
Fig. 5 is a cross-sectional view of the elastic web material.
Fig. 6 is a plan view of an absorbent core according to the present disclosure.

### DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
The expression "% by weight" (weight percent) otherwise referred to as "wt%", here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants, diaper holders and liners, sanitary napkins and the like, as well as surgical bandages and sponges. Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. Disposable absorbent articles can include a liquid pervious top sheet, a back sheet joined to the top sheet, and an absorbent core positioned and held between the top sheet and the back sheet. The top sheet is operatively permeable to the liquids that are intended to be held or stored by the absorbent article, and the back sheet may or may not be substantially impervious or otherwise operatively impermeable to the intended liquids. The absorbent article may also include other components, such as liquid wicking layers, liquid intake layers, liquid distribution layers, transfer layers, barrier layers, wrapping layers and the like, as well as combinations thereof. Disposable absorbent articles and the components thereof can operate to provide a body-facing surface and a garment-facing surface.

The "absorbent medium" or "absorbent core" or "absorbent body" is the absorbent structure disposed between the top sheet and the back sheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. **It** is common to combine cellulosic fluff pulp with superabsorbent polymers in an absorbent material.

"Acquisition and distribution layer", "ADL" or "surge management portion" refers to a sublayer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the top sheet and the retention portion.

The term "adhesive" as used herein is intended to refer to any suitable hot melt, water or solvent borne adhesive that can be applied to a surface of a film layer in the required pattern or network of adhesive areas to form the film-nonwoven laminate of the present invention. Accordingly, suitable adhesives include conventional hot melt adhesives, pressure-sensitive adhesives and reactive adhesives (i.e., polyurethane).

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used herein, an "airformed web" refers to a material comprising cellulosic fibers such as those from fluff pulp that have been separated, such as by a hammermilling process, and then deposited on a porous surface without a substantial quantity of binder fibers present.

Airfelt materials used as the absorbent core in many diapers, for example, are a typical example of an airformed material.

As used herein, an "airlaid web" is a fibrous structure formed primarily by a process involving deposition of air-entrained fibers onto a mat, typically with binder fibers present, and typically followed by densification and thermal bonding. In addition to traditional thermally bonded airlaid structures (those formed with non-tacky binder material present and substantial thermally bonded), the scope of the term "airlaid" according to the present invention can also include coform, which is produced by combining air-entrained dry, dispersed cellulosic fibers with meltblown synthetic polymer fibers while the polymer fibers are still tacky. Further, an airformed web to which binder material is subsequently added can be considered within the scope of the term "airlaid" according to the present invention. Binder can be added to an airformed web in liquid form (e. g., an aqueous solution or a melt) by spray nozzles, direction injection or impregnation, vacuum drawing, foam impregnation, and so forth. Solid binder particles can also be added by mechanical or pneumatic means.

"Application-Force" is the force that a wearer of caretaker might encounter while donning the absorbent article. The Application-Force is derived from a two cycle Hip Hoop Test.

The term "back sheet" refers to a material forming the outer cover of the absorbent article. The back sheet prevents the exudates contained in the absorbent structure from wetting articles such as bedsheets and overgarments which contact the disposable absorbent article. The back sheet may be a unitary layer of material or may be a composite layer composed of multiple components assembled side-by-side or laminated. The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials.

The term "bio-based" may refer to a component of an absorbent article that can be produced or is derived from a renewable resource, as defined herein.

As used herein, the "body-facing" or "bodyside" surface means that surface of the article or component which is intended to be disposed toward or placed adjacent to the body of the wearer during ordinary use, while the "outward", "outward-facing" or "garment-side" surface is on the opposite side, and is intended to be disposed to face away from the wearer's body during ordinary use. Such outward surface may be arranged to face toward or placed adjacent to the wearer's undergarments when the absorbent article is worn.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "breathable" refers to layers, preferably films or elastic laminates, having a water vapor transmission rate (WVTR) of at least 300 grams/m² - 24 hours.

"Carded web" refers to webs that are made from staple fibers that are sent through a combing or carding unit, which opens and aligns the staple fibers in the machine direction to form a generally machine direction-oriented fibrous nonwoven web. The web is then bonded by one or more of several known bonding methods. Bonding of nonwoven webs may be achieved by a number of methods; powder bonding, wherein a powdered adhesive or a binder is distributed through the web and then activated, usually by heating the web and adhesive with hot air; pattern bonding, wherein heated calendar rolls or ultrasonic bonding equipment are used to bond the fibers together, usually in a localized bond pattern, though the web can be bonded across its entire surface if so desired; through-air bonding, wherein air which is sufficiently hot to soften at least one component of the web is directed through the web; chemical bonding using, for example, latex adhesives that are deposited onto the web by, for example, spraying; and consolidation by mechanical methods such as needling and hydroentanglement.

As used herein, the term "cellulosic" is meant to include any material having cellulose as a major constituent, and specifically comprising at least 50 percent by weight cellulose or a cellulose derivative. Thus, the term includes cotton, typical wood pulps, nonwoody cellulosic fibers, cellulose acetate, cellulose triacetate, rayon, thermomechanical wood pulp, chemical wood pulp, debonded chemical wood pulp, milkweed, or bacterial cellulose.

As used herein "channels" are fluid distribution means within the absorbent core adapted to favour exudate flow there along and are typically intended to exclude embossing patterns or ducts formed by compression from the meaning thereof and rather include structures that are substantially free of absorbent material instead of comprising compacted absorbent material. Channels herein are formed by joining upper and lower layers of a core wrap as will be described in more detail hereinbelow. Channels preferably comprise recessed regions forming visible conduits or passages typically extending along the longitudinal axis of the core and having a depth in a direction perpendicular to said longitudinal axis. By "visible" it is herein intended clearly visible by naked eye. Typically the channels have a width generally greater than 1mm, preferably from 5mm to 50mm, more preferably from 6mm to 40mm, more preferably from 8mm to 30mm, even more preferably from greater than 8mm to less than 25mm.

"Chassis" refers to a foundational constituent of an absorbent article upon which the remainder of the structure of the article is built up or overlaid, e.g., in a diaper, the structural elements that give the diaper the form of briefs or pants when configured for wearing, such as a back sheet, a top sheet, or a combination of a top sheet and a back sheet.

"Comprise", "comprising", and "comprises" and "comprised of" as used herein are synonymous with "include", "including", "includes" or "contain", "containing", "contains" and are inclusive or openended terms that specifies the presence of what follows e.g. component and do not exclude or preclude the presence of additional, non-recited components, features, element, members, steps, known in the art or disclosed therein.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

Further, the diaper can comprise "containment flaps" or "barrier cuffs". The containment flaps are generally thought to be particularly well suited for the containment of fecal matter and to prevent the lateral flow of liquid waste until such time as the liquid waste can be absorbed by the absorbent article. Many constructions of containment flaps are known. Such containment flaps generally comprise a proximal edge, intended to be attached to the absorbent article, and an opposite distal edge which is generally not attached to the absorbent article along at least a portion of its length. An elastic member is generally located adjacent the distal edge to assist in maintaining the containment flap in an upright condition and in maintaining a sealing relationship between the distal edge of the containment flap and the body of a wearer during use. The elastic member is generally located between two layers of material so that the elastic does not come into contact with the body of a wearer. The containment flaps may be manufactured from a wide variety of materials such as polypropylene, polyester, rayon, nylon, foams, plastic films, formed films, and elastic foams. A number of manufacturing techniques may be used to manufacture the containment flaps. For example, the containment flaps may be woven, non-woven, spunbonded, carded, cast, blown or the like.

The diaper can comprise leg containment gaskets. Leg "containment gaskets" help prevent leakage of bodily exudates when the wearer exerts compressive forces on the absorbent article. In particular, the stiffness of the leg containment gaskets prevents twisting and bunching of the leg openings of the absorbent article which can lead to leaks. **In** addition, the elasticity and conformability of the leg containment gaskets ensures that the bodyfacing surface of the leg containment gaskets provides an adequate seal against the body of the wearer. The physical properties of the leg containment gaskets, such as the thickness and stiffness, also function to space the bodyside liner, outer cover and absorbent core away from the wearer's body when in use. As such, void volume is created between the wearer's body and the bodyside liner and absorbent core of the absorbent article to help contain body exudates.

"Continuous" means that the described structure is a closed-loop structure. The continuous structure may be unitary, i.e., a one-piece structure, or may be made up of individual elements suitably joined together to form a closed-loop.

"Conventional hot-melt adhesive" means a formulation that generally comprises several components. These components typically include one or more polymers to provide cohesive strength (e.g., aliphatic polyolefins such as poly (ethylene-co-propylene) copolymer; ethylene vinyl acetate copolymers; styrene-butadiene or styrene- isoprene block copolymers; etc.); a resin or analogous material (sometimes called a tackifier) to provide adhesive strength (e.g., hydrocarbons distilled from petroleum distillates; rosins and/or rosin esters; terpenes derived, for example, from wood or citrus, etc.); perhaps waxes, plasticizers or other materials to modify viscosity (i.e., flowability) (examples of such materials include, but are not limited to, mineral oil, polybutene, paraffin oils, ester oils, and the like); and/or other additives including, but not limited to, antioxidants or other stabilizers. A typical hot-melt adhesive formulation might contain from about 15 to about 35 weight percent cohesive strength polymer or polymers; from about 50 to about 65 weight percent resin or other tackifier or tackifiers; from more than zero to about 30 weight percent plasticizer or other viscosity modifier; and optionally less than about 1 weight percent stabilizer or other additive. It should be understood that other adhesive formulations comprising different weight percentages of these components are possible.

"Decitex" also known as Dtex is a measurement used in the textile industry for measuring yarns or filaments. 1 Decitex = 1 gram per 10,000 meters. In other words, if 10,000 linear meters of a relaxed yarn or filament weights 500 grams that yarn or filament would have a decitex of 500.

The term "density" or "concentration" when referring to the absorbent material, in particular the SAP, of a layer, refers to the amount of the absorbent material divided by the surface area of the layer over which the absorbent material is spread out.

"Discontinuous bonding pattern" as used herein refers to a pattern of bonding areas, in particular bonding areas between layers, whereby at least in at least one region the layers are not bonded. A discontinuous bonding pattern may comprise a connected bonding area or multiple disconnected bonding areas. A discontinuous bonding pattern further may comprise a connected bonding area comprising a number of holes, where the layers are not bonded, preferably according to a regular pattern, or it may comprise discrete disconnected bonding areas, e.g. a point bonded pattern which comprises a plurality of separate bonding points surrounded by unbonded areas or a line-bonded pattern which comprises a plurality of separate bonding lines alternated by unbonded areas, preferably according to a regular pattern.

The term "disposable" may be used herein to describe absorbent articles and other products which are not intended to be laundered or otherwise restored or reused as an absorbent article or product (i.e., they are intended to be discarded after use and, preferably, to be recycled, composted or otherwise disposed of in an environmentally compatible manner).

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

The term "elasticized" or "elastified" refers to a material, layer, or substrate that is naturally non-elastic, but which has been rendered elastic by, for example, suitably joining an elastic material, layer, or substrate thereto.

As used herein, the term "elastic resistance" describes an elastic force that tends to resist an applied tensile force causing a material provided therewith to tend to contract to an untensioned configuration in response to a stretching force.

"Elongation" means the ratio of the extension of a material to the length of the material prior to the extension (expressed in percent). "Extension" means the change in length of a material due to stretching (expressed in units of length).

As used herein the term "extensible" means elongatable in at least one direction, but not necessarily recoverable.

The term "film" refers to a sheet-like material wherein the length and width of the material far exceed the thickness of the material. Typically, films have a thickness of about 0.5 mm or less.

The term "flexible" refers to materials which are compliant and which will readily conform to the general shape and contours of the wearer's body.

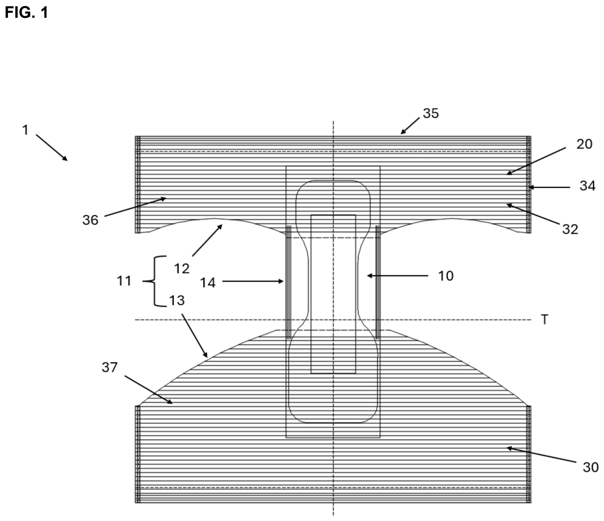

The term "fully extended state" refers to the state in which the absorbent article is laid flat, with all elastic components extended to the point where the material in which they are incorporated is maximally extended in the direction the materials are designed to elongate when in use, but without causing damage or permanent deformation to any of the article's components. For the purpose of dimensional measurements, the "fully extended state" may correspond to a 2D technical drawing, prepared by a designer for production purposes, showing a top plan view of the article in an opened-up and laid-flat state, with all elastic components extended as described above and as shown for example in Figure 1. **In** case of discrepancy, the 2D technical drawing takes precedence over the physical extension of the article.

The term "high-absorbency material" refers to materials that are capable of absorbing at least 10 times their own weight in liquid. The high-absorbency material may comprise absorbent gelling materials, such as superabsorbent polymers. Superabsorbent polymers are water-swellable, water-insoluble organic or inorganic materials capable of absorbing at least about 20 times their own weight of an aqueous solution containing 0.9 weight percent of sodium chloride. Absorbent gelling materials can be natural, synthetic and modified natural polymers and materials. In addition, the absorbent gelling materials can be inorganic materials, such as silica gels, or organic compounds such as cross-linked polymers. The term "cross-linked" refers to any means for effectively rendering normally water-soluble materials substantially water insoluble but swellable. Such means can include, for example, physical entanglement, crystalline domains, covalent bonds, ionic complexes and associations, hydrophilic associations, such as hydrogen bonding, and hydrophobic associations or Van der Waals forces. Examples of synthetic absorbent gelling material polymers include the alkali metal and ammonium salts of poly(acrylic acid) and poly (methacrylic acid), poly(acrylamides), poly(vinyl ethers), maleic anhydride copolymers with vinyl ethers and alphaolefins, poly(vinyl pyrrolidone), poly(vinylmorpholinone), poly(vinyl alcohol), and mixtures and copolymers thereof. Further polymers suitable for use in the absorbent structure include natural and modified natural polymers, such as hydrolyzed acrylonitrile-grafted starch, acrylic acid grafted starch, methyl cellulose, carboxymethyl cellulose, hydroxypropyl cellulose, and the natural gums, such as alginates, xanthan gum, locust bean gum and the like. Mixtures of natural and wholly or partially synthetic absorbent polymers can also be used. Synthetic absorbent gelling materials typically are xerogels which form hydrogels when wetted. The term "hydrogel", however, has commonly been used to also refer to both the wetted and unwetted forms of the material. The high-absorbency material may be in any of a wide variety of geometric forms. As a general rule, it is preferred that the high-absorbency material be in the form of discrete particles. However, the high-absorbency material may also be in the form of fibres, flakes, rods, spheres, needles, spiral or semi-spiral, cubic, rod-like, polyhedral, or the like. Conglomerates of particles of high-absorbency material may also be used. The high-absorbency material may be present in the absorbent core in an amount of from about 5 to about 100 weight percent and desirably from about 30 to about 100 weight percent based on the total weight of the absorbent core. The distribution of the high-absorbency material within the different portions of the absorbent core can vary depending upon the intended end use of the absorbent core. The high-absorbency material may be arranged in a generally discrete layer within the matrix of hydrophilic fibres. Alternatively, the absorbent core may comprise a laminate of fibrous webs and high-absorbency material or other suitable means of maintaining a high-absorbency material in a localized area.

Although the term "hook" or "hook element" is used herein when referring to a fastener element, it is understood that fastener elements are not limited to hook-like shapes and may be in the shape of mushrooms, tees, hooks, multi-lobed hooks, pins, projections or other shapes as desired. Such shapes may act as fastener elements as formed or may be post formed to make them suitable as a fastener for use in touch fastener.

The term "hydrophilic" describes fibers or the surfaces of fibers which are wetted by the aqueous liquids in contact with the fibers. The degree of wetting of the materials can, in turn, be described in terms of the contact angles and the surface tensions of the liquids and materials involved. The term "wettable" is meant to refer to a fiber which exhibits a liquid, such as water, synthetic urine, or a 0.9 weight percent aqueous saline solution, in air contact angle of less than 90°, whereas "hydrophobic" or "non-wettable" describes fibers having contact angles equal to or greater than 90°.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

As used herein, "Substantially liquid impermeable" as used herein refers to a barrier (or wicking layer) that comprises a material that will substantially prevent the passage of liquids through it. It should be noted that the material can be breathable so as to permit the passage of gasses or vapors, but not of liquids, or the material can be configured so as to prevent the passage of liquids as well as gasses or vapors. The degree of impermeability of a material is typically measured by the hydrostatic head.

"Substantially absorbent particulate polymer material impermeable" as used herein refers to an edge barrier (or wicking layer) that comprises a material that will substantially prevent the passage of absorbent particulate polymer material through it.

"Integral" is used to refer to various portions of a single unitary element rather than separate structures bonded to or placed with or placed near one another.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

The term "laid-flat state" is intended to refer to the article when it is flattened into a plane or is substantially flattened into a plane and is used in contrast to when the article otherwise positioned, such as when the article is folded or shaped in or for use by a wearer.

"Laminate" refers to elements being attached together in a layered arrangement.

The use of the term "layer" can refer, but is not limited, to any type of substrate, such as a woven web, nonwoven web, films, laminates, composites, elastomeric materials, or the like. A layer can be liquid and air permeable, permeable to air but impermeable to liquids, impermeable both to air and liquid, or the like. When used in the singular, it can have the dual meaning of a single element or a plurality of elements.

"Longitudinal" is a direction running parallel to the maximum linear dimension of the article.

"Loop" or "loop material" may include any structure or material that is suitable for mating with a fastener element, such as for example a hook element. The loop may be formed from a woven, nonwoven, foam, screen or mesh substrate or combinations thereof.

The term "machine direction" or "MD" may be used herein to refer to the direction of material flow through a manufacturing process. In addition, relative placement and movement of material can be described as flowing in the machine direction through a process from upstream in the process to downstream in the process. The term "cross direction" or "CD" may be used herein to refer to a direction that is generally perpendicular to the machine direction.

The term "meltblown fibers" means fibers formed by extruding a molten thermoplastic material through a plurality of fine, usually circular, die capillaries as molten threads or filaments into a high velocity gas stream (e.g. air) which attenuates the filaments of molten thermoplastic material to reduce their diameter, which may be to microfiber diameter. In general, meltblown fibers have an average fiber diameter of up to about 10 microns. After the fibers are formed, the meltblown fibers are carried by the high velocity gas stream and are deposited on a collecting surface to form a web of randomly disbursed meltblown fibers.

The term "nonelastic" or "inelastic" refers to any material which does not fall within the definition of "elastic" above, typically by not comprising elastic materials such as elastic web materials described herein.

The term "nonwoven fabric or web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

"Pre-strain" refers to the strain imposed on an elastic or elastomeric material prior to combining it with another element of the elastomeric laminate or the absorbent article. Pre-strain is determined by the following equation Pre-strain = ((extended length of the elastic-relaxed length of the elastic)/relaxed length of the elastic)* 100.

Further, the term "projection" or "pin" may refer to an element that by itself or through a post forming operation can function as a fastener element. Further, the terms "fastener element" and "fastening elements" are used interchangeably herein.

"Proximal" and "Distal" refer respectively to the location of an element relatively near to or far from the longitudinal or lateral centerline of a structure (e.g., the proximal edge of a longitudinally extending element is located nearer to the longitudinal axis than the distal edge of the same element is located relative to the same longitudinal axis).

"Pulp fluff" or "fluff pulp" refers to a material made up of cellulose fibers. The fibers can be either natural or synthetic, or a combination thereof. The material is typically lightweight and has absorbent properties.

As used herein the term "sheet" or "sheet material" refers to woven materials, nonwoven webs, polymeric films, polymeric scrim-like materials, and polymeric foam sheeting.

The term "spunbond fibers" refers to fibers formed by extruding molten thermoplastic polymers as filaments or fibers from a plurality of relatively fine, usually circular, capillaries of a spinneret, and then rapidly drawing the extruded filaments by an eductive or other well-known drawing mechanism to impart molecular orientation and physical strength to the filaments. The average diameter of spunbond fibers is typically in the range of from 15-60 µm or higher. The spinneret can either be a large spinneret having several thousand holes per meter of width or be banks of smaller spinnerets, for example, containing as few as 40 holes.

The term "spunbond meltblown spunbond" (SMS) nonwoven fabric as used herein refers to a multi-layer composite sheet comprising a web of meltblown fibers sandwiched between and bonded to two spunbond layers. A SMS nonwoven fabric can be formed in-line by sequentially depositing a first layer of spunbond fibers, a layer of meltblown fibers, and a second layer of spunbond fibers on a moving porous collecting surface. The assembled layers can be bonded by passing them through a nip formed between two rolls that can be heated or unheated and smooth or patterned. Alternately, the individual spunbond and meltblown layers can be pre-formed and optionally bonded and collected individually such as by winding the fabrics on wind-up rolls. The individual layers can be assembled by layering at a later time and bonded together to form a SMS nonwoven fabric. Additional spunbond and/or meltblown layers can be incorporated in the SMS fabric, for example spunbond-meltblown-meltblown-spunbond (SMMS), etc.

"Staple fibers" refer to commercially available fibers having diameters ranging from less than about 0.001 mm to more than about 0.2 mm; they come in several different forms such as short fibers ranging from about 10 to 50 mm in length and long fibers with a length higher than 50 mm, preferably up to 100 mm.

By "stretch", it is meant that the material has the ability to extend beyond its original size in at least one dimension when subjected to a tensile force (i. e., tension) applied in the direction of that dimension, without breaking the material. An extension of for example 50% means that the material with an initial length of 100mm has reached a length of 150mm. "Stretch" may be unidirectional, bidirectional, or multi- directional. The specific "stretch" properties of a material may vary along any of the stretch vectors. The term can include elastic materials, as well as nonwovens that can be inherently extensible, but not necessarily in an elastic manner. Such nonwovens can be made to behave in an elastic manner by bonding them to elastic films.

Use of the term "substrate" includes, but is not limited to, woven or nonwoven webs, porous films, ink permeable films, paper, composite structures, or the like.

Superabsorbent materials suitable for use in the present invention are known to those skilled in the art, and may be in any operative form, such as particulate form, fibers and mixtures thereof. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g. saline with 0.9 wt % NaCl). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof. The hydrogel-forming polymers may be lightly crosslinked to render the material substantially water insoluble. Crosslinking may, for example, be by irradiation or covalent, ionic, Van der Waals, or hydrogen bonding. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article of the present invention in an amount up to about 60% by weight. Typically, the superabsorbent material, when present, will be included in an amount of about 5% to about 40% by weight, based on the total weight of the absorbent layer.

"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 µm, preferably between 300 to 600 µm, more preferably between 400 to 500 µm.

"Tension" includes a uniaxial force tending to cause the extension of a body or the balancing force within that body resisting the extension.

As used herein, the term "thermoplastic" is meant to describe a material that softens when exposed to heat and which substantially returns to its original condition when cooled to room temperature.

The term "topsheet" or "top sheet" refers to a liquid permeable material sheet forming the inner cover of the absorbent article and which in use is placed in direct contact with the skin of the wearer. The top sheet is typically employed to help isolate the wearer's skin from liquids held in the absorbent structure. The top sheet may be joined to portions of the backsheet, the absorbent core, the barrier leg cuffs and/or any other layers as is known to those of ordinay skill in the art. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g. urine or menstrual fluid. The inner coversheet may further be different in different parts of the absorbent article. The top sheet fabrics may be composed of a substantially hydrophobic material, and the hydrophobic material may optionally be treated with a surfactant or otherwise processed to impart a desired level of wettability and hydrophilicity.

As used herein, the term "transverse" or "lateral" refers to a line, axis, or direction which lies within the plane of the absorbent article and is generally perpendicular to the longitudinal direction. "Ultrasonic welding" refers to a technology which joins two materials by melting them with heat generated from ultrasonic oscillation and then laminating them together, such that the molten materials flow and fill the gap between the two unaffected portions of the two materials, respectively. Upon cooling and shaping, the two materials are joined together.

As used herein, the term "water-swellable, water-insoluble" is meant to refer to a material that, when exposed to an excess of water, swells to its equilibrium volume but does not dissolve into the solution. As such, a water-swellable, water-insoluble material generally retains its original identity or physical structure, but in a highly expanded state, during the absorption of the water and, thus, must have sufficient physical integrity to resist flow and fusion with neighbouring particles. Embodiments of the articles according to the disclosure will now be described. It is understood that technical features described in one or more embodiments maybe combined with one or more other embodiments without departing from the intention of the disclosure and without generalization therefrom.

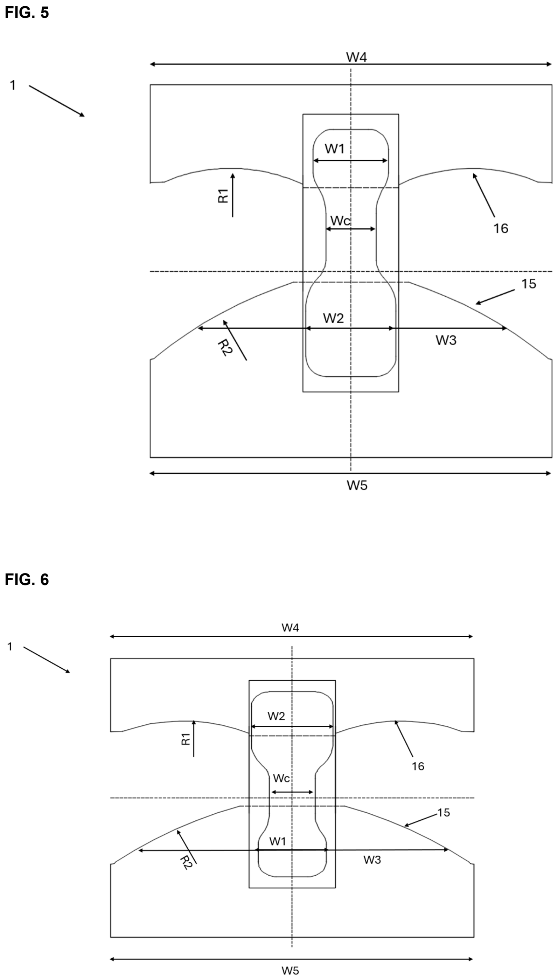

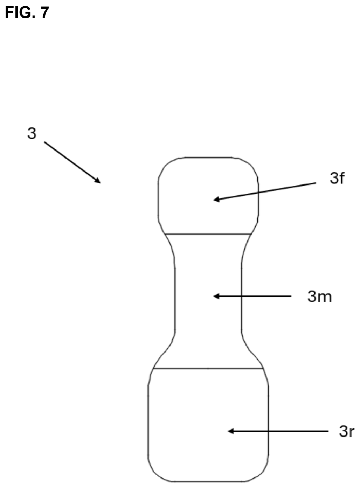

In a preferred embodiment, as shown in Fig. 1, the absorbent article (1) comprises a front waist belt (20) and a rear waist belt (30). The front waist belt (20) generally is the portion of the article intended to be worn on the front side of the wearer, while the rear waist belt (30) generally is intended to be worn on the back side of the wearer. An absorbent insert (10) is joined, at least partially, to the front and rear waist belts (20, 30). The absorbent insert (10) may serve as the primary absorbent structure of the article, capturing and retaining fluids. The absorbent insert (10) comprises a top sheet (2), a back sheet (4), and an absorbent core (3). The top sheet (2) typically is the layer closest to the wearer's skin, allowing fluids to pass through to the absorbent core (3). The back sheet (4) typically forms the outer layer of the insert, preventing fluids from leaking out of the article. The absorbent core (3) is positioned, at least partially, between the top sheet (2) and the back sheet (4) and has as primary function the absorption and retention of fluids. The absorbent core (3) comprises a front core portion (3f), a middle core portion (3m), and a rear core portion (3r), as shown in figure 6.

Each of the front and rear waist belts (20, 30) comprises an outer nonwoven layer (33), an inner nonwoven layer (31), and an elastic material (32) sandwiched, at least partially, between the outer and inner nonwoven layers (31, 33). The outer and inner nonwoven layers (31, 33) may provide a soft and cloth-like feel, while the elastic material (32) may provide stretch and conformability for a secure fit. The front and rear waist belts (20, 30) are joined together at a pair of side seams (34) to form a waist opening (WO) with a waist edge (35) extending in the transverse direction (T). The side seams (34) may create the pant-like configuration of the article, allowing it to be pulled up and down like regular underwear.

The absorbent article (1) further comprises two leg openings (LO), each being bordered by a leg edge (11). The leg edge (11) has a front leg edge (12) being located generally on the front waist belt (20), a rear leg edge (13) being located generally on the rear waist belt (30), and a crotch leg edge (14) generally located between the front leg edge (12) and the rear leg edge (13). The leg openings (LO) are designed to fit comfortably around the wearer's legs, and may provide a secure barrier against leakage. The rear leg edge (13) comprises at least a first convex curved segment (15). This convex curvature may contribute to a snug and comfortable fit around the wearer's buttocks.

The absorbent core (3) has a first width (W1) and a second width (W2) in fully extended state, wherein the second width (W2) is larger than the first width (W1). The absorbent core (3) may further comprise an upper core wrap layer and a lower core wrap layer wherein said core wrap layers are preferably nonwoven webs. The absorbent material of said absorbent core may be contained within at least one core wrap substrate enclosing said absorbent material. The top core wrap layer and bottom core wrap layer may form part of the same substrate or may be 2 separate substrates. Furthermore, the top layer of said core wrap may be adhered to a bottom layer of said core wrap forming one or more channels substantially free of said absorbent material. Such adhesion may advantageously provide core stability and for example reduce the risk of sagging. This adhesion may be provided by any means known in the art to bond core wrap layers together, for example with adhesive or mechanical bonding. Advantageously, the presence of channels within an absorbent core may further improve the liquid distribution and liquid acquisition in order to better use the entire surface area and absorption capacity of the front core portion, middle core portion and rear core portion.

The outer nonwoven layer (33) may comprise a first corrugated web and the inner nonwoven layer (32) may comprise a second corrugated web, provided with ridges alternating with depressions and connected to each other at the depressions, wherein said elastic material is entrapped between said first corrugated web and said second corrugated web. The outer nonwoven layer (33) and inner nonwoven layer (32) can be attached to each other by means of ultrasonic bonding, thermal bonding, adhesive bonding or any combination thereof. Advantageously, this may result in an elastic laminate with uniform and regular pleating which is soft to touch. Preferably, the elastic material entrapped between said first and second corrugated webs may comprise a thread-like element and/or a ribbon-like element, or a plurality of thread-like elements and/or a plurality of ribbon-like elements, or their combinations. Lower tensions can be applied to said elastic threads. Therefore, it is possible that the thread-like element, or rather, the elastic thread may have a count less than or equal to 50 tex, even from 10 tex to 40 tex. This may allow significant reduction of the production costs, and also may simplify the manipulation of the elastic threads under tension, significantly reducing the possibility that they may break.

In a first example according to said preferred embodiment, the absorbent core (3) comprises an absorbent core crotch width (Wc) being less than the first width (W1) and the second width (W2). The absorbent core crotch width (Wc) represents the narrowest width of the absorbent core (3) in the crotch region. By having the absorbent core crotch width (Wc) less than the first width (W1) and the second width (W2), the absorbent article (1) may provide improved comfort and fit in the crotch region, while maintaining sufficient absorbent capacity in the front core portion (3f) and the rear core portion (3r). Preferably, as shown in Figures 5 and 6, the absorbent core crotch width (Wc) is positioned in the middle core portion (3m) and is measured at a point where the absorbent core (3) is narrowest in the middle core portion (3m). By positioning the absorbent core crotch width (Wc) in the middle core portion (3m), the absorbent article (1) may provide a contoured fit that minimizes bulk and may enhance comfort in the crotch region. The narrower width in the middle core portion (3m) may allow the absorbent article (1) to conform to the wearer's anatomy, and may reduce the risk of chafing and irritation.

In a second example, as shown in Figure 1, according to the preferred embodiment and/or the first example, the front leg edge (12) comprises at least a first concave curved segment (16). This concave curvature may allow the front leg edge (12) to better conform to the shape of the wearer's leg, and may provide enhanced comfort and freedom of movement. The concave curved segment (16) may also contribute to a more aesthetically pleasing design. Preferably, the at least first concave curved segment (16) has a first radius (R1), and the at least first convex curved segment (15) has a second radius (R2) in fully extended state, wherein R2 is larger than R1, as illustrated in Figures 5 and 6. The relationship between the first radius (R1) and the second radius (R2) may enable the absorbent article (1) to provide a balance between a secure fit and wearer comfort. The larger second radius (R2) of the convex curved segment (15) in the rear leg edge (13) may help to contain bodily fluids, while the smaller first radius (R1) of the concave curved segment (16) in the front leg edge (12) may allow for greater freedom of movement.

In a third example, as shown in Figure 5, according to said preferred embodiment and/or any of the previous examples, the absorbent core (3) has a first width (W1) and a second width (W2) in fully extended state. The first width (W1) is positioned in the front core portion (3f) and is measured at a point where the absorbent core (3) is widest in the front core portion (3f). The second width (W2) is positioned in the rear core portion (3r) and is measured at a point where the absorbent core (3) is widest in the rear core portion (3r). By positioning the first width (W1) and the second width (W2) as described, the absorbent core (3) may provide enhanced absorbent capacity in the rear core portion (3r) where the absorbent article (1) is most likely to receive fluid.

In a fourth example, as shown in Figure 6, according to said preferred embodiment and/or any of examples 1 to 2, the absorbent core (3) has a first width (W1) and a second width (W2) in fully extended state. The second width (W2) is positioned in the front core portion (3f) and is measured at a point where the absorbent core (3) is widest in the front core portion (3f). The first width (W1) is positioned in the rear core portion (3r) and is measured at a point where the absorbent core (3) is widest in the rear core portion (3r). By positioning the first width (W1) and the second width (W2) as described, the absorbent core (3) may provide enhanced absorbent capacity in the front core portion (3f) where the absorbent article (1) is most likely to receive fluid when the wearer is a male subject.

In a fifth example, as shown in Figure 5, according to said third example, the first convex curved segment (15) has a second radius (R2), and the absorbent core (3) has a second width (W2) at the rear core portion (3r) in fully extended state. The ratio of R2 and W2 is between about 1.5 and 7.5, more preferably between about 2 and 7, more preferably between about 3 and 6.5, more preferably between about 3.5 and 6, and even more preferably between about 4 and 6. This specific range of the R2/W2 ratio may further optimize the fit and containment properties of the absorbent article (1) by providing a balance between the curvature of the rear leg edge (13) and the width of the absorbent core (3).

In a sixth example, according to the fifth example, the absorbent article (1) also comprises a rear leg profile width (W3), measured according to the method disclosed herein. The ratio of W3 and W2 is between about 2 and 6, more preferably between about 2.5 and 5, and most preferably between about 3 and 4. This range of the W3/W2 ratio may ensure that the rear leg profile provides adequate coverage and containment while maintaining a comfortable fit around the wearer's leg.

In a seventh example, as shown in Figure 6, according to said fourth example, the first concave curved segment (16) has a first radius (R1), and the absorbent core (3) has a second with (W2) at the front core portion (3f) in fully extended state. The ratio of R1 and W2 is between about 1,5 and 4; more preferably between about 1,75 and 2,5; even more preferably between about 1,8 and 2,25. This specific range of the R1/W2 ratio may further optimize the fit and containment properties of the absorbent article (1) by providing a balance between the curvature of the front leg edge (12) and the width of the absorbent core (3).

In an eight example, according to any of the preferred embodiment and/or previous examples, the ratio of W2 and W1, wherein W2 represents the second width of the absorbent core (3) in the rear core portion (3r) and W1 represents the first width of the absorbent core (3) in the front core portion (3f), is between about 1.03 and 1.5, preferably between about 1.1 and 1.4, even more preferably between 1.15 and 1.3, and most preferably between 1.18 and 1.25. By controlling the ratio between the first width (W1) and the second width (W2), the absorbent article (1) may be able to provide a balance between absorbent capacity and fit. A wider rear core portion (3r) may provide enhanced absorbent capacity, while a narrower front core portion (3f) may improve comfort and reduces bulk.

In an ninth example, according to any of the preferred embodiment and/or previous examples, the front waist belt (20) has a width W4 and the rear waist belt (30) has a width W5 and wherein the ratio of W4 and W2 is between 3 and 10, preferably between about 3 and 9, more preferably between about 3 and 8, or wherein the ratio of W5 and W2 is between about 3 and 10, preferably between about 3 and 9, more preferably between about 3 and 8. This may provide for better fit and comfort in the front waist region or back waist region in relation to the positioning of the widest part of the absorbent core.

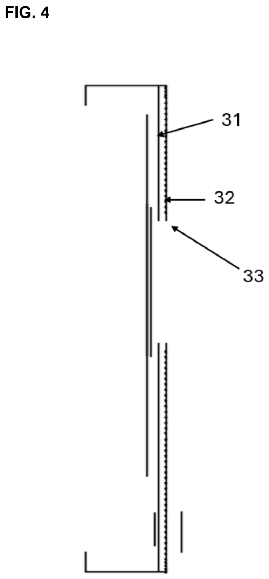

In a tenth example, according to any of the preferred embodiment and/or previous examples, the elastic material (32) that is sandwiched, at least partially, between the outer nonwoven layer (33) and the inner nonwoven layer (31) of the front and rear waist belts (20, 30) can be selected from the group consisting of an elastic film, elastic strands, and elastic fibers and combinations thereof. The use of an elastic film may provide a uniform and consistent stretch force, while the use of elastic strands may allow for targeted and customizable elasticity. Combinations of elastic films and elastic strands can be used to achieve a desired balance of stretch, conformability, and cost. The elastic material (32) may contribute to the overall fit and conformability of the absorbent article (1), allowing it to move with the wearer while maintaining a secure and leak-resistant seal. As shown in Figure 4, the outer nonwoven layer (33), the elastic material (32) and the inner nonwoven layer (31) may form an elastic laminate. Preferably, the elastic material (32) partially overlaps the rear core portion (3r). This configuration may provide enhanced fit and containment in the rear portion of the absorbent article (1), and may ensure that the absorbent core (3) remains in close contact with the wearer's body for optimal fluid absorption and retention. Further preferably, the elastic material (32) partially overlaps the front core portion (3f). This configuration may provide enhanced fit and containment in the front portion of the absorbent article (1), and may ensure that the absorbent core (3) remains in close contact with the wearer's body for optimal fluid absorption and retention. The partial overlap of the elastic material (32) with the front core portion (3f) and the rear core portion (3r) may allow for a more secure and comfortable fit, may minimize bulk and may prevent the absorbent core (3) from shifting or bunching during use. This configuration may also contribute to improved leakage prevention by maintaining close contact between the absorbent core (3) and the wearer's body.

In an eleventh example, according to the preferred embodiment and/or any of the previous examples, the front waist belt (20) comprises a first plurality of elastic strands (36), and the rear waist belt (37) comprises a second plurality of elastic strands (37). Each of the first and second pluralities has an Average-Strand-Spacing from 0.25 mm to 4 mm as measured according to the test method set out herein. By controlling the spacing of the elastic strands, the absorbent article (1) can provide a comfortable and secure fit around the wearer's waist while allowing for breathability and flexibility. Preferably, each of the first and second pluralities of elastic strands (36, 37) has an Average-Dtex from 10 to 400 as measured according to the test method set out herein. The Dtex value of the elastic strands may further optimize the fit of the absorbent article (1) by providing a balance between the elastic force and the comfort of the waist belts. Preferably, at least a portion of each of the first and second pluralities of elastic strands (36, 37) has a Pressure-Under-Strand of from 689 to 6895 Pa (0.1 to 1 psi) as measured according to the test method set out herein. Defining the pressure under the strand may ensure that the elastic strands provide a consistent and effective fit around the wearer's waist. Preferably, each of the first and second plurality of elastic strands (36, 37) has an Average-Pre-Strain from 100% to 250% as measured according to the test method set out herein. The pre-strain of the elastic strands may determine the amount of tension that is applied to the waist belts and may contribute to the overall fit and containment properties of the absorbent article (1).

In a twelfth example, according to the preferred embodiment and/or the eleventh example, the absorbent article (1) has an Application-Force of from 900 gf to 1600 gf as measured according to the test method set out herein. The application force is a measure of the force required to apply the absorbent article (1) to the wearer, and this range may ensure that the article can be easily applied and removed while providing a secure fit.

In a thirteenth example, according to the preferred embodiment and/or any of examples 1 to 10, wherein the elastic material (32) comprises elastic fibers comprising a thermoplastic elastomer and said fibers running substantially parallel to each other and oriented along a transversal axis (x). Advantageously, the elastic properties are derived directly from the engineered structure of the front and/or rear waist belts, rather than from separate elastic films or strands merely sandwiched between nonwoven layers. This integrated approach simplifies the overall belt construction and can lead to superior performance and tactile characteristics. In the context of a diaper pant, the transverse axis (x) typically corresponds to the circumferential direction of the article when worn. This orientation is highly advantageous for waist belts, as it provides the necessary elastic stretch for a comfortable and conforming fit around the wearer's waist. These specifically oriented elastic fibers are often produced via a specialized manufacturing process known as "direction spinning", as described in documents such as WO2025/168762 A1 and WO2025/168761 A1. In this process, thermoplastic elastomer polymers are extruded through a spinneret to form single filaments which are then mechanically drawn (e.g., via a godet roll system) without a diffusor, thereby maintaining their parallel alignment and machine direction orientation. When this nonwoven web is subsequently used for a waist belt, its machine direction is aligned with the circumferential direction of the pant. The technical advantages of utilizing such an inherently elastic nonwoven for the waist belts are substantial. Firstly, it provides a highly uniform distribution of elastic force across the entire width of the belt. Unlike discrete strands, which can concentrate force at specific points and potentially cause red marking on the skin, these abundant, thin, parallel elastic fibers distribute tension more evenly, leading to significantly enhanced wearer comfort. Secondly, the resulting waist belts are inherently thinner and less bulky in their relaxed state, contributing to improved discretion under clothing and a more premium feel. Thirdly, these materials often exhibit high MD elasticity with low CD elongation, which is ideal for pant waistbands: strong stretch for easy donning and circumferential fit, combined with vertical stability to prevent excessive sagging. Furthermore, the manufacturing process for such materials can often be optimized for material efficiency, reducing overall basis weight and environmental impact. This embodiment thus represents a sophisticated material solution that elevates the performance and comfort of the absorbent article's waist belt.

### METHODS OF THE PRESENT DISCLOSURE

### Rear leg profile width (W3)

Using a ruler calibrated against a certified NIST ruler and accurate to 0.5 mm, measure the distance between the two rear leg edges to the nearest 0.5 mm at the widest point of the back core portion in fully extended state of the absorbent article.

In case the back core portion comprises a straight section running substantially parallel to the longitudinal center axis (y) and wherein said straight section comprises the widest point of the back core portion, measure W3 at the center in longitudinal direction of said straight section, as shown for example in figure 5.

### General Sample Preparation

The General Sample Preparation is intended to be used for methods that do not have specific sample preparation instructions within the method itself.

When collecting a specimen for testing, the specimen must contain a plurality of elastic strands and/or an elastic material, elastic scrim, elastic ribbons, elastic strips, etc. In situations where the elastic material and/or elastic strands is not fully secured within the sample, the test specimen must be obtained in a way that elastic material and/or elastic strands within the test region of the specimen are as they were intended and not altered as a result of collection of the specimen. If the elastic material or any elastic strands release, creep or become separated within or from the laminate, the specimen is discarded and a new specimen prepared. And, depending on the method, the portion or area of the stranded elastomeric laminate that should be tested will include a plurality of elastic strands between an area of first and second nonwovens, excluding any cut window (such as an elastic-free zone or area overlapping with the core or center chassis, and excluding any seams joining multiple article components together. Certain methods, however, may require testing of the absorbent article component, including cut windows and seams (e.g., Hip Hoop Test).

For pants, remove the side panels where they are attached to the chassis and separate the side panels at the side seams. Identify the elastic material that transverses the entire width of the panel. Identify the longitudinally distal most edge of the elastic material or elastic strand (closest to the waist edge) and the longitudinally proximal most edge of the elastic material or elastic strand (closest to the leg edge) determine the midpoint between the distal most elastic strand or elastic material edge and the proximal most elastic strand or elastic material edge. Cut a 40 mm wide strip laterally across the entire panel centered at the midpoint. Repeat for each front and rear side panel that contains elastic material and/or elastic strands.

### Average-Strand-Spacing

Using a ruler calibrated against a certified NIST ruler and accurate to 0.5 mm, measure the distance between the two distal strands within a section to the nearest 0.5 mm, and then divide by the number of strands in that section - 1
- Average-Strand-Spacing = d/(n-1) where n>1

### Pressure-Under-Strand (also referred to as Average-Pressure-Under-Strand)

Defined as the average pressure imparted by each individual elastic strand of a section under specific conditions. These conditions are defined as (refer to FIG. 21):
- The section is pulled to a Stress of 7 gf/mm (within a consumer preferred range of stresses as determined experimentally)
- The section is pulled over a cylinder whose circumference is defined as a Representative-Circumference
Where:
- Pressure-Under-Strand (psi) = 1.422 * Strand-Force / (2 * Representative-Radius * Average-Strand-Diameter)
- Representative-Radius (mm) = Representative-Circumference / (2 * pi)
- Representative-Circumference(mm) = 460 mm
- Stress (gf/mm) = (Summation of Strand-Forces within a section) / (Section-Width)
- Section-Width (mm) = (Number of Elastics in the section) * Average-Strand-Spacing (mm)
- Strand-Force (gf) = Strand-Strain (%) * 0.046875 * Average-Dtex
- Strand-Strain (%) = strain in each elastic strand within a section
- Average-Strand-Diameter (mm) = 2 * sqrt (Strand-Cross-Sectional-Area / pi)
- Strand-Cross-Sectional-Area (mm2) = Average-Dtex / Strand-Density / 10,000
- Strand-Density (g/cc) = 1.15 g/cc (industry standard for PolyUrethaneUrea based spandex elastics)
- Dtex (g/10,000m) = Standard textile unit of measure. Dtex is weight in grams for 10,000m of the material
- Average-Pre-Strain = Amount of stretch in elastic strands in a section prior to combining with substrate layer(s).
- Maximum-Strain = Average-Pre-Strain. This is the maximum amount of strain each section can be pulled to. It cannot exceed the Average-Pre-Strain.
- Maximum-Section-Force = Summation of each strand in the section pulled to the Maximum-Strain.

### Average-Decitex (Average-Dtex)

The Average-Decitex Method is used to calculate the Average-Dtex on a length-weighted basis for elastic fibers present in an entire article, or in a specimen of interest extracted from an article. The decitex value is the mass in grams of a fiber present in 10,000 meters of that material in the relaxed state. The decitex value of elastic fibers or elastomeric laminates containing elastic fibers is often reported by manufacturers as part of a specification for an elastic fiber or an elastomeric laminate including elastic fibers. The Average-Dtex is to be calculated from these specifications if available. Alternatively, if these specified values are not known, the decitex value of an individual elastic fiber is measured by determining the cross-sectional area of a fiber in a relaxed state via a suitable microscopy technique such as scanning electron microscopy (SEM), determining the composition of the fiber via Fourier Transform Infrared (FT-IR) spectroscopy, and then using a literature value for density of the composition to calculate the mass in grams of the fiber present in 10,000 meters of the fiber. The manufacturer-provided or experimentally measured decitex values for the individual elastic fibers removed from an entire article, or specimen extracted from an article, are used in the expression below in which the length-weighted average of decitex value among elastic fibers present is determined.

The lengths of elastic fibers present in an article or specimen extracted from an article is calculated from overall dimensions of and the elastic fiber pre-strain ratio associated with components of the article with these or the specimen, respectively, if known. Alternatively, dimensions and/or elastic fiber pre-strain ratios are not known, an absorbent article or specimen extracted from an absorbent article is disassembled and all elastic fibers are removed. This disassembly can be done, for example, with gentle heating to soften adhesives, with a cryogenic spray (e.g. Quick-Freeze, Miller-Stephenson Company, Danbury, CT), or with an appropriate solvent that will remove adhesive but not swell, alter, or destroy elastic fibers. The length of each elastic fiber in its relaxed state is measured and recorded in millimeters (mm) to the nearest mm.

### Calculation of Average-Dtex

For each of the individual elastic fibers f of relaxed length Z, and fiber decitex value *dᵢ* (obtained either from the manufacturer's specifications or measured experimentally) present in an absorbent article, or specimen extracted from an absorbent article, the Average-Dtex for that absorbent article or specimen extracted from an absorbent article is defined as: $Average - Dtex = \frac{{\sum }_{i = 1}^{n} \left({L}_{i}\times {d}_{i}\right)}{{\sum }_{i = 1}^{n} {L}_{i}}$ where n is the total number of elastic fibers present in an absorbent article or specimen extracted from an absorbent article. The Average-Dtex is reported to the nearest integer value of decitex (grams per 10 000 m). If the decitex value of any individual fiber is not known from specifications, it is experimentally determined as described below, and the resulting fiber decitex value(s) are used in the above equation to determine Average-Dtex.

### Experimental Determination of Decitex Value for a Fiber

For each of the elastic fibers removed from an absorbent article or specimen extracted from an absorbent article according to the procedure described above, the length of each elastic fiber *Lₖ* in its relaxed state is measured and recorded in millimeters (mm) to the nearest mm. Each elastic fiber is analyzed via FT-IR spectroscopy to determine its composition, and its density *ρₖ* is determined from available literature values. Finally, each fiber is analyzed via SEM. The fiber is cut in three approximately equal locations perpendicularly along its length with a sharp blade to create a clean cross-section for SEM analysis. Three fiber segments with these cross-sections exposed are mounted on an SEM sample holder in a relaxed state, sputter coated with gold, introduced into an SEM for analysis, and imaged at a resolution sufficient to clearly elucidate fiber cross-sections. Fiber cross-sections are oriented as perpendicular as possible to the detector to minimize any oblique distortion in the measured cross-sections. Fiber cross-sections may vary in shape, and some fibers may consist of a plurality of individual filaments. Regardless, the area of each of the three fiber cross-sections is determined (for example, using diameters for round fibers, major and minor axes for elliptical fibers, and image analysis for more complicated shapes), and the average of the three areas *aₖ* for the elastic fiber, in units of micrometers squared (µm²), is recorded to the nearest 0.1 µm². The decitex *dₖ* of the kth elastic fiber measured is calculated by: ${d}_{k} = 10 000 m \times {α}_{k} \times {ρ}_{k} \times {10}^{- 6}$ where *dₖ* is in units of grams (per calculated 10,000 meter length), *aₖ* is in units of µm², and *ρₖ* is in units of grams per cubic centimeter (g/cm³). For any elastic fiber analyzed, the experimentally determined *Lₖ* and *dₖ* values are subsequently used in the expression above for Average-Dtex.

### Average-Pre-Strain

The Average-Pre-Strain of a specimen are measured on a constant rate of extension tensile tester (a suitable instrument is the MTS Insight using Testworks 4.0 Software, as available from MTS Systems Corp., Eden Prairie, MN) using a load cell for which the forces measured are within 1% to 90% of the limit of the cell. Articles are conditioned at 23 °C ± 2 C° and 50% ± 2% relative humidity for 2 hours prior to analysis and then tested under the same environmental conditions.

Program the tensile tester to perform an elongation to break after an initial gage length adjustment. First raise the cross head at 10 mm/min up to a force of 0.05N. Set the current gage to the adjusted gage length. Raise the crosshead at a rate of 100 mm/min until the specimen breaks (force drops 20% after maximum peak force). Return the cross head to its original position. Force and extension data is acquired at a rate of 100 Hz throughout the experiment.

Set the nominal gage length to 40 mm using a calibrated caliper block and zero the crosshead. Insert the specimen into the upper grip such that the middle of the test strip is positioned 20 mm below the grip. The specimen may be folded perpendicular to the pull axis, and placed in the grip to achieve this position. After the grip is closed the excess material can be trimmed. Insert the specimen into the lower grips and close. Once again, the strip can be folded, and then trimmed after the grip is closed. Zero the load cell. The specimen should have a minimal slack but less than 0.05 N of force on the load cell. Start the test program.

From the data construct a Force (N) verses Extension (mm). The Average-Pre-Strain is calculated from the bend in the curve corresponding to the extension at which the nonwovens in the elastic are engaged. Plot two lines, corresponding to the region of the curve before the bend (primarily the elastics), and the region after the bend (primarily the nonwovens). Read the extension at which these two lines intersect, and calculate the % Pre-Strain from the extension and the corrected gage length. Record as % Pre-strain 0.1%. Calculate the arithmetic mean of three replicate samples for each elastomeric laminate and Average-Pre-Strain to the nearest 0.1%.

## Claims

1. An absorbent article (1), preferably in the form of a pant, comprising:
- a front waist belt (20);
- a rear waist belt (30);
- an absorbent insert (10) joined to the front and rear waist belts (20, 30); said absorbent insert (10) comprising a top sheet (2), a back sheet (4) and an absorbent core (3), wherein said absorbent core (3) comprises a front core portion (3f), a middle core portion (3m) and a rear core portion (3r);
wherein the front and rear waist belts (20, 30) each comprise an outer nonwoven layer (33), an inner nonwoven layer (31), and an elastic material (32) sandwiched between said outer and inner nonwoven layers (31, 33);
the front and rear waist belts (20, 30) joined together at a pair of side seams (34) to form a waist opening (WO) with a waist edge (35) extending in transverse direction (T);
wherein said absorbent article (1) further comprises two leg openings (LO) each being bordered by a leg edge (11) having a front leg edge (12) being located generally on the front waist belt (20), a rear leg edge (13) being located generally on the rear waist belt (30) and a crotch leg edge (14) generally located between the front leg edge (12) and the rear leg edge (13),
**characterised in that** said rear leg edge (13) comprising at least a first convex curved segment (15) and said absorbent core (3) has a first width (W1) and a second width (W2), wherein said second width (W2) is larger than said first width (W1).

2. An absorbent article (1) according to Claim 1, wherein said absorbent core (3) comprises an absorbent core crotch width (Wc) being less than W1 and W2.

3. An absorbent article (1) according to Claim 2, wherein said absorbent core crotch width (Wc) is positioned in said middle core portion (3m) and is measured at a point where the absorbent core (3) is narrowest in the middle core portion (3m).

4. An absorbent article (1) according to any of the preceding claims, wherein said front leg edge (12) comprises at least a first concave curved segment (16).

5. An absorbent article (1) according to Claim 4, wherein said at least first concave curved segment (16) has a first radius (R1) and said at least first convex curved segment (15) has a second radius (R2), wherein R2 is larger than R1.

6. An absorbent article (1) according to any of the preceding Claims, wherein said first width (W1) is positioned in said front core portion (3f) and is measured at a point where the absorbent core (3) is widest in the front core portion (3f) and said second width (W2) is positioned in said rear core portion (3r) and is measured at a point where the absorbent core (3) is widest in the rear core portion (3r).

7. An absorbent article (1) according to any of Claims 1 to 5, wherein said second width (W2) is positioned in said front core portion (3f) and is measured at a point where the absorbent core (3) is widest in the front core portion (3f) and said first width (W1) is positioned in said rear core portion (3r) and is measured at a point where the absorbent core (3) is widest in the rear core portion (3r).

8. An absorbent article (1) according to Claim 6, wherein a ratio of R2 and W2 is between about 1,5 and 7,5; more preferably between about 2 and 7; more preferably between about 3 and 6,5; more preferably between about 3,5 and 6; even more preferably between about 4 and 6.

9. An absorbent article (1) according to Claim 8, wherein said article (1) comprises a rear leg profile width (W3), measured according to the method disclosed herein, and wherein a ratio of W3 and W2 is between about 2 and 6, more preferably between about 2,5 and 5; most preferably between about 3 and 4.

10. An absorbent article (1) according to Claim 7, wherein a ratio of R1 and W2 is between about 1,5 and 7,5; more preferably between about 1,5 and 6; more preferably between about 1,5 and 4; more preferably between about 1,75 and 2,5 ; even more preferably between about 1,8 and 2,25.

11. An absorbent article (1) according to any of the preceding Claims, wherein the ratio of W2 and W1 is between about 1,03 and 1,5; preferably between about 1,1 and 1,4; even more preferably between 1,15 and 1,3; most preferably between 1,18 and 1,25.

12. An absorbent article (1) according to any of the preceding Claims, wherein the front waist belt (20) has a width W4 and the rear waist belt (30) has a width W5 and wherein the ratio of W4 and W2 is between about 3 and 10, preferably between about 3 and 9, more preferably between about 3 and 8, or wherein the ratio of W5 and W2 is between about 3 and 10, preferably between about 3 and 9, more preferably between about 3 and 8.

13. An absorbent article (1) according to any of the preceding Claims, wherein the elastic material (32) is selected from the group consisting of an elastic film, elastic strands, elastic fibers and combinations thereof.

14. An absorbent article (1) according to any of the preceding Claims, wherein the elastic material (32) partially overlaps the rear core portion (3r).

15. An absorbent article (1) according to any of the preceding Claims, wherein the elastic material (32) partially overlaps the front core portion (3f).

16. An absorbent article (1) according to any of the preceding Claims, wherein said front waist belt (20) comprises a first plurality of elastic strands (36) and wherein said rear waist belt (37) comprises a second plurality of elastic strands, each of the first and second pluralities have an Average-Strand-Spacing from 0,25 mm to 4 mm as measured according to the test method set out herein.

17. An absorbent article (1) according to Claim 16, wherein each of the first and second pluralities of elastic strands (36, 37) have an Average-Dtex from 10 to 400 as measured according to the test method set out herein.

18. An absorbent article (1) according to any of Claims 16 to 17, wherein at least a portion of each of the first and second pluralities of elastic strands (36, 37) has a Pressure-Under-Strand of from 689 to 6895 Pa (0,1 to 1 psi) as measured according to the test method set out herein.

19. An absorbent article (1) according to any of Claims 16 to 18, wherein each of the first and second plurality of elastic strands (36, 37) has an Average-Pre-Strain from 100% to 250% as measured according to the test method set out herein.

20. An absorbent article (1) according to any of Claims 16 to 19, wherein said article (1) has an Application-Force of from 900 gf to 1600 gf as measured according to the test method set out herein.

21. An absorbent article (1) according to any of 1 to 15, wherein the elastic material (32) comprises elastic fibers comprising a thermoplastic elastomer and said fibers running substantially parallel to each other and oriented along a transversal axis (x).
